# EUROPEAN PATENT APPLICATION

(11) **EP 2 009 002 A1**
(43) Date of publication of application: **31.12.2008**
(21) Application number: 07012155.3
(22) Date of filing: 21.06.2007
(51) Int. Cl.: C07D 233/90, C07D 471/04

(54) **New process for the manufacture of 1H-imidazo [4,5-c]-quinoline ring systems**

(71) Applicant: INSTITUT NATIONAL DE LA SANTE ET DE LA RECHERCHE MEDICALE (INSERM), 75013 Paris (FR)
(72) Inventor: Galons, Hervé, 75014 Paris (FR); Gug, Fabienne, 95230 Soisy (FR)
(74) Representative: Leszczynski, André

(57) **Abstract**

The present invention relates to a new synthetic route to manufacture 1H-imidazo[4,5-c]-quinoline ring systems and to new corresponding intermediates. Said new intermediates are biaryl aminonitrile compounds of formula (II)

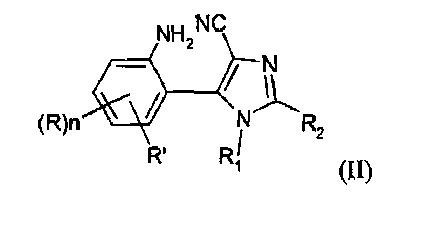

## Description

The present invention relates to a method for preparing 1H-imidazo[4,5-c]-quinoline ring systems derivatives.

Most of said compounds are known. They can, in particular, be used as antiviral agents, or some of them as antineoplasic agents.

More particularly, Imiquimod or 1-isobutyl-1H-imidazo[4,5-c]quinolin-4-amine, pertaining to said derivatives family, is an immune response modifier that is used in several cutaneous diseases and namely useful for treating viral infections, such as genital warts, basal cell carcinoma (BCC) and squamous cell carcinoma (SCC). Imiquimod is disclosed in EP 145 340 and in the equivalent US 4,689,338. It has the following structure:

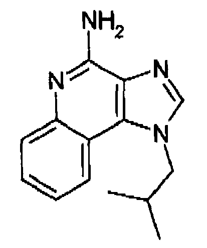

Resiquimod or 4-amino-2-(ethoxy-methyl)-α,α-dimethyl-1H-imidazo[4,5-c]quinoline-1-ethanol, also pertaining to said derivatives family, is also useful as an antiviral agent. Resiquimod is disclosed in WO 92/15582. It has the following structure:

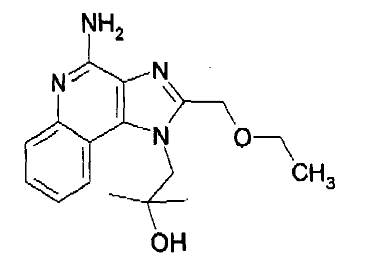

Several methods are known in the art to manufacture 1H-imidazo[4,5-c]-quinoline ring systems, including Imiquimod and Resiquimod.

In most cases the amino group at the 4-position of the desired final compound is introduced in the last steps of the sequence. Some documents are even entirely dedicated to the synthetic step consisting in the introduction of said amino group.

Most of these syntheses involve a step with the cyclisation of a quinoline derivative, having for example amino groups at the 3- and 4- positions (for example in EP 145 340) or a nitro group at the 3-position and an amino group a the 4- position (for example in EP 425 306), to afford a 1H-imidazo[4,5-c]quinoline to which the amino group at the 4- position is to be further introduced. 8 to 9 steps may be required to achieve the desired compound.

Alternative preparation processes are also reported for example in WO97/48704 involving a quinoline bearing a nitro group and a tetrazole ring fused to it.

All the previous described synthetic route, in addition to their high number of steps, present the drawback to need the use of nitro derivatives which are hard, and even risky to handle.

To the knowledge of the inventors, only the two following documents deal with the synthesis of Imiquimod using another kind of cyclisation, not involving a quinoline derivative.

US2005/0165236 discloses a process for the preparation of Imiquimod implementing an intramolecular aromatic nucleophilic substitution between the NH₂ group and the X group in a compound of formula 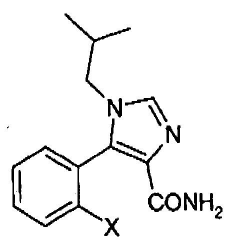 wherein X is a leaving group.

In H.Yoshioka and al, Chem. Pharm. Bull. 44(4) 709-714 (1996) the tricyclic imidazoquinoline ring is accessed using two types of pyrido-annelation based on the thermal electrocyclic reactions of a so-called azahexatriene system involving the imidazole 4,5- bond for the constructions.

However these two last described methods present drawbacks that are major woodblocks against the industrialization. Strong bases (eg *n*-butyllithium and *tert-*butyllithium) are required. Moreover said two processes also require a large number of steps and subsequently offer no practical advantages compared to the classical quinoline based synthetic routes.

Therefore a need still exists to find new synthetic routes which do not present the herabove mentioned drawbacks.

Unexpectedly, the inventors discovered that a short synthesis of 1H-imidazo[4,5-c]-quinoline ring systems is possible by implementing a Suzuki-Miyaura coupling between a highly reactive imidazole derivative and a pinacol 2-(aminophenyl)boronate derivative, providing a new synthetic intermediate which in turn is able to provide by cyclisation a 1H-imidazo[4,5-c]-quinoline ring systems derivative.

This approach offers several advantages. One of the major advantages lies in the fact that the number of synthetic steps is decreased which provides an economical interest. Moreover the starting materials are easily available. It is also to be noticed that the conditions are mild and reaction under pressure is avoided. Work-ups are easy: All the intermediates can be isolated by crystallization. Moreover, the construction of the 1H-imidazo[4,5-c]-quinoline ring systems derivative by a convergent synthesis can allow the synthesis of new derivatives hard to prepare by the previously reported procedures.

Accordingly, the present invention relates, according to a first aspect, to a new compound of formula (II) 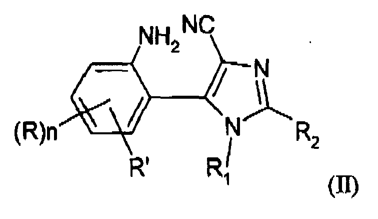 wherein
**R₁** represents:
- an hydrogen atom,
- a (C₁-C₁₀)alkyl group,
- a phenyl group,
- a benzyl group,
- a (phenyl) ethyl group,
- a (C₂-C₁₀)alkenyl group,
- a tetrahydrofuranylmethyl group,
- R₁₁,
- W-R₁₁,
- W-Y-R₁₁, or
- W-R₅,
where the (C₁-C₁₀)alkyl group can possibly be substituted by one or two identical or different groups chosen among an hydroxy group, a (C₁-C₄)alkoxy group, a hydroxy(C₁-C₄)alkoxy group, a (C₃-C₆)cycloalkyl group, a (C₂-C₄)alkanoyloxy (C₁-C₆)alkyl group, a benzoyloxy(C₁-C₆)alkyl group, a (C₂-C₁₀)alk-1-ynyl group, a tetrahydropyranyl group, a (C₁-C₄)alkoxy(C₁-C₄)alkyl group or a 2-, 3- or 4-pyridyl group, where the (C₂-C₁₀)alkenyl group can be possibly substituted by one or two identical or different groups chosen among a (C₁-C₄)alkyl group, a (C₃-C₆)cycloalkyl group optionally substituted by a (C₁-C₄) alkyl group, and
where the tetrahydrofuranyl group can be possibly substituted by one substituent selected among a hydroxy group and a (C₁-C₄)hydroxyalkyl group,
**R₂** represents:
- an hydrogen atom,
- a trifluoromethyl group,
- a (C₁-C₈)alkyl group,
- a mercapto group,
- a (C₁-C₄)alkylthio group,
- a benzyl group,
- a (phenyl)ethyl group,
- a phenyl group,
where the (C₁-C₈)alkyl group can possibly be substituted by one to three identical or different groups chosen among a phenyl group, a (C₁-C₄)alkoxy group, a (C₁-C₄)alkoxy(C₁-C₄)alkyl group, a hydroxy(C₁-C₄)alkyl group, a halo(C₁-C₄)alkyl group, a (C₁-C₄)alkylamido, an amino group or an amino group substituted independently by a (C₁-C₄)alkyl group or a hydroxy(C₁-C₄)alkyl group, an azide group, a chlorine atom, a hydroxy group, a 1-morpholino group, a 1-pyrrolidino group or a (C₁-C₄)alkylthio group, where the phenyl group of the benzyl, the (phenyl)ethyl or the phenyl group in R₁ or R₂ can be possibly substituted by one or two substituents selected from the group consisting of a (C₁-C₄)alkyl group, a (C₁-C₄)alkoxy group, a (C₁-C₄)alkyl(C₂-C₄)alkoxy group or a halogen atom,
**R** independently represents:
- a (C₁-C₁₀)alkoxy group,
- an halogen group, with the proviso that R is different from a bromine or a iodine atom except when the R group is in the para position to the NH₂ group on the benzene ring,
- a (C₁-C₄)alkyl group,
- a hydroxy group, or
- a trifluoromethyl group,
n is an integer from 0 to 2, and
**R'** represents H or when n is 0 or 1 **R'** is H or may be selected from the group consisting of:
- Z-Ar,
- Z-Ar'-Y-R₁₁,
- Z-Ar'-W-Y-R₁₁,
- Z-Ar'-R₅, and
- Z-Ar'-W-R₅;
where Ar is selected from the group consisting of aryl and heteroaryl both of which can be unsubstituted or can be substituted by one or more substituents independently selected from the group consisting of alkyl, alkenyl, alkoxy, methylenedioxy, haloalkyl, haloalkoxy, halogen, nitro, hydroxy, hydroxyalkyl, mercapto, cyano, carboxy, formyl, aryl, aryloxy, arylalkoxy, heteroaryl, heteroaryloxy, heteroarylalkoxy, heterocyclyl, heterocyclylalkyl, amino, alkylamino, and dialkylamino;
Ar' is selected from the group consisting of arylene and heteroarylene both of which can be unsubstituted or can be substituted by one or more substituents independently selected from the group consisting of alkyl, alkenyl, alkoxy, haloalkyl, haloalkoxy, halogen, nitro, hydroxy, hydroxyalkyl, mercapto, cyano, carboxy, formyl, aryl, aryloxy, arylalkoxy, heteroaryl, heteroarylox, heteroarylalkoxy, heterocyclyl, heterocyclylalkyl, amino, alkylamino, and dialkylamino;
**W** is selected from the group consisting of alkylene, alkenylene, alkynylene, arylene, heteroarylene, and heterocyclylene wherein the alkylene, alkenylene, and alkynylene groups can be optionally interrupted or terminated with arylene, heteroarylene, or heterocyclylene, and optionally interrupted by one or more -O- groups;
**Y** is selected from the group consisting of:
-O-
-S(O)₀₋₂-, -S(O)₂-N(R₈)-, -C(R₆)-,
-C(R₆)-O-, -O-C(R₆)-, -O-C(O)-O-,
-N(R₈)-O-, -C(R₆)-N(R₈)-,
-O-C(R₆)-N(R₈)-, -C(R₆)-N(OR₉)-, 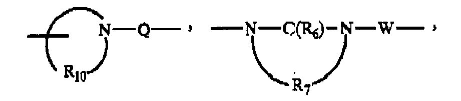 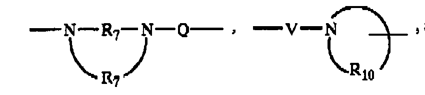 and 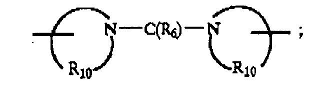 **Z** is selected from the group consisting of a bond, alkylene, alkenylene, and alkynylene;
**R₁₁** is selected from the group consisting of hydrogen, alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl wherein the alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl groups can be unsubstituted or substituted by one or more substituents independently selected from the group consisting of alkyl, alkoxy, hydroxyalkyl, haloalkyl, haloalkoxy, halogen, nitro, hydroxy, mercapto, cyano, aryl, aryloxy, arylalkyleneoxy, heteroaryl, heteroaryloxy, heteroarylalkyleneoxy, heterocyclyl, amino, alkylamino, dialkylamino, (dialkylamino)alkyleneoxy, and in the case of alkyl, alkenyl, alkynyl, and heterocyclyl, oxo;
**R₅** is selected from the group consisting of: 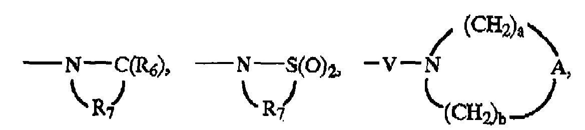 and 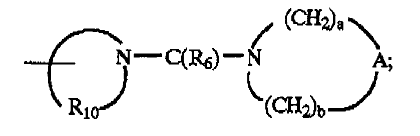 each **R₆** is independently selected from the group consisting of =O and =S;
each **R₇** is independently C₂₋₇ alkylene;
**R₈** is selected from the group consisting of hydrogen, alkyl, alkoxyalkylenyl, hydroxyalkylenyl, heteroarylalkylenyl and arylalkylenyl;
**R₉** is selected from the group consisting of hydrogen and alkyl;
each **R₁₀** is independently C₃₋₈ alkylene;
**A** is selected from the group consisting of -O-, -C(O)-, -S(O)₀₋₂-, -N(Q-R₁₁)-, -CH₂-, and -N(R₁₁)-;
**Q** is selected from the group consisting of a bond, -C(R₆)-, -C(R₆)-C(R₆)-, -S(O)₂-, -C(R₆)-N(R₈)-W'-, -S(O)₂-N(R₈)-, -C(R₆)-O-, -C(R₆)-S- and -C(R₆)-N(OR₉)-;
**V** is selected from the group consisting of -C(R₆)-, -O-C(R₆)-, -N(R₈)-C(R₆)-, and -S(O)₂-;
**W'** is selected from the group consisting of a bond, -C(O)-, and -S(O)₂-; and
a and b are independently integers from 1 to 6 with the proviso that a+b is ≤7;
or a pharmaceutically acceptable acid addition salt thereof.

According to a particular aspect of the invention, the compounds as defined above are useful as synthetic intermediates.

According to a particular embodiment of the first aspect, the present invention relates to a new compound of formula (II) as defined above
wherein
**R₁** represents
- an hydrogen atom,
- a (C₁-C₁₀)alkyl group,
- a (C₁-C₆)hydroxyalkyl group,
- an acyloxyalkyl group wherein the acyloxy moiety is alkanoyloxy of two to about four carbon atoms or benzoyloxy, and the alkyl moiety contains one to about six carbon atoms,
- a benzyl group,
- a (phenyl)ethyl group,
- a phenyl group,
where the phenyl group of the benzyl, the (phenyl)ethyl or the phenyl group can be possibly substituted by one or two substituents selected from the group consisting of a (C₁-C₄)alkyl group, a (C₁-C₄)alkoxy group, or a halogen atom,
**R₂** represents
- an hydrogen atom,
- a (C₁-C₈)alkyl group,
- a benzyl group,
- a (phenyl)ethyl group,
- a phenyl group,
where the phenyl group of the benzyl, the (phenyl)ethyl or the phenyl group can be possibly substituted by one or two substituents selected from the group consisting of a (C₁-C₄)alkyl group, a (C₁-C₄)alkoxy group or a halogen atom,
**R** independently represents
- a (C₁-C₄)alkoxy group,
- an halogen group, with the proviso that R is different from a bromine or a iodine atom except when the R group is in the para position to the NH₂ group on the benzene ring,
- a (C₁-C₄)alkyl group,
- n is an integer from 1 to 2, and
- **R'** is an hydrogen atom,
- or a pharmaceutically acceptable acid addition salt thereof.

According to a particular preferred embodiment of the first aspect, the present invention relates to a new compound of formula (11) wherein R₁ represents an isobutyl group, R₂ and R' are hydrogen atoms and n is 0.

According to a further particular preferred embodiment of the first aspect, the present invention relates to a new compound of formula (II) wherein R₁ is a 2-methyl, 2-hydroxypropyl group, R₂ is an ethoxymethyl group, R' is a hydrogen atom and n is 0.

According to a second aspect, the present invention provides a process for the preparation of a compound of formula (II) as defined above comprising at least the step of reacting compound of formula 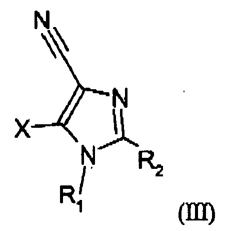 wherein R₁, R₂ are as defined above, and X is a diazonium group, an other leaving group such as triflates, e.g.trifluoromethylsulfonate or an halogen atom and preferably a bromine or a iodine atom, by coupling, in the presence of a catalyst, with a compound of formula 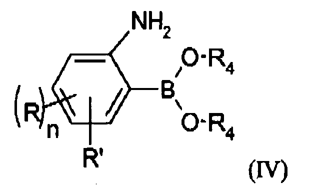 wherein R, R' and n are as defined above, and
wherein R₄ represents an hydrogen atom, an alkyl group and where the two R₄ groups can also be bound together to form a 5- or 6-membered ring 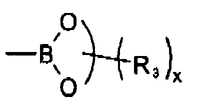 where represents a (C₂-C₄)alkylene possibly substituted, x is an integer from 1 to 4 and R₃ independently represents an hydrogen atom or a (C₁-C₄)alkyl group.
The 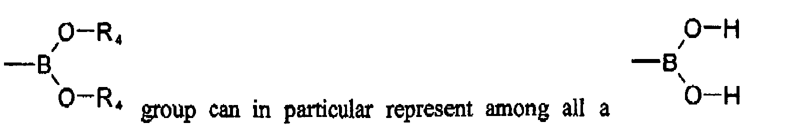 group, a

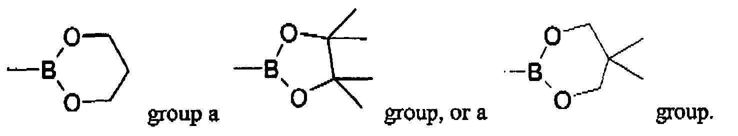

It will be readily apparent to those skilled in the art that changing the ester can be easily done without generating a new invention

According to a third aspect, the present invention provides a new process for the preparation of preparing 1H-imidazo[4,5-c]-quinoline ring systems derivatives of formula (I) 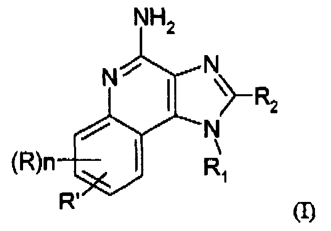 wherein R₁, R₂, R, R' and n are as defined above,
comprising at least the step of reacting a compound of formula (II) as defined above, by cyclisation under basic conditions.

The compounds of formula (I) can comprise one or more asymmetrical carbon atoms. They can thus exist in the form of enantiomers or of diastereoisomers. These enantiomers, diastereoisomers, as their mixtures, including the racemic mixtures form part of the definition of formula (I).

The compounds of formula (I) may exist in the form of free bases or of addition salts with pharmaceutically acceptable acids. Compounds of formula (I) containing an alcohol can be also used as esters in particular esters from amino-acids.

Suitable pharmaceutically acceptable salts of compounds of formula (I) include base addition salts and where appropriate acid addition salts. Suitable physiologically acceptable base addition salts of compounds of formula (I) include alkali metal or alkaline metal salts such as sodium, potassium, calcium, and magnesium salts, and ammonium salts, formed with amino acids (e.g. lysine and arginine) and organic bases (e.g. procaine, phenylbenzylamine, ethanolamine diethanolamine and N-methyl glucosamine).

Suitable acid addition salts may be formed with organic acid and inorganic acids e.g. hydrochloric acid.

The compounds of formula (I) and/or salts thereof may also form solvates (e.g. hydrates) and the definition of compounds of formula (I) includes all such solvates.

As used herein, the terms "alkyl", "alkenyl", "alkynyl" and the prefix "alk-" are inclusive of both straight chain and branched chain groups and of cyclic groups, i. e. cycloalkyl and cycloalkenyl. Unless otherwise specified, these groups contain from 1 to 20 carbon atoms, with alkenyl groups containing from 2 to 20 carbon atoms, and alkynyl groups containing from 2 to 20 carbon atoms. In some embodiments, these groups have a total of up to 10 carbon atoms, up to 8 carbon atoms, up to 6 carbon atoms, or up to 4 carbon atoms. Cyclic groups can be monocyclic or polycyclic and preferably have from 3 to 10 ring carbon atoms. Exemplary cyclic groups include cyclopropyl, cyclopropylmethyl, cyclopentyl, cyclohexyl, adamantyl, and substituted and unsubstitutedbornyl, norbornyl and norbornenyl.

Unless otherwise specified "alkylene", "alkenylene" and "alkynylene" are the divalent forms of the "alkyl", "alkenyl" and "alkynyl" groups defined above. Likewise, "alkylenyl", "alkenylenyl" and "alkynylenyl" are the divalent forms of the "alkyl", "alkenyl" and "alkynyl" groups defined above. For example, an arylalkylenyl group comprises an alkylene moiety to which an aryl group is attached.

The term "haloalkyl" is inclusive of groups that are substituted by one or more halogen atoms, including perfluorinated groups. This is also true of other groups that include the prefix "halo-". Examples of suitable haloalkyl groups are chloromethyl, trifluoromethyl, and the like.

The term "aryl" as used herein includes carbocyclic aromatic rings or ring systems. Examples of aryl groups include phenyl, naphthyl, biphenyl, fluorenyl and indenyl.

The term "heteroatom" refers to the atoms O, S, or N.

The term "heteroaryl" includes aromatic rings or ring systems that contain at least one ring heteroatom (e. g., O, S, N). Suitable heteroaryl groups include furyl, thienyl, pyridyl, quinolinyl, isoquinolinyl, indolyl, isoindolyl, triazolyl, pyrrolyl, tetrazolyl, imidazolyl, pyrazolyl, oxazolyl, thiazolyl, benzofuranyl, benzothiophenyl, carbazolyl, benzoxazolyl, pyrimidinyl, benzimidazolyl, quinoxalinyl, benzothiazolyl, naphthyridinyl, isoxazolyl, isothiazolyl, purinyl, quinazolinyl, pyrazinyl,1-oxidopyridyl, pyridazinyl, triazinyl, tetrazinyl, oxadiazolyl, thiadiazolyl, and so on.

The term "heterocyclyl" includes non-aromatic rings or ring systems that contain at least one ring heteroatom (e. g., 0, S, N) and includes all of the fully saturated and partially unsaturated derivatives of the above mentioned heteroaryl groups. Exemplary heterocyclic groups include pyrrolidinyl, tetrahydrofuranyl, morpholinyl, thiomorpholinyl, piperidinyl, piperazinyl, thiazolidinyl, imidazolidinyl, isothiazolidinyl, tetrahydropyranyl, quinuclidinyl, homopiperidinyl, homopiperazinyl, and the like.

The terms "arylene", "heteroarylene" and "heterocyclylene" are the divalent forms of the "aryl", "heteroaryl" and "heterocyclyl" groups defined above. Likewise, "arylenyl", "heteroarylenyl" and "heterocyclylenyl" are the divalent forms of the "aryl", "heteroaryl" and "heterocyclyl" groups defined above. For example, an alkylarylenyl group comprises an arylene moiety to which an alkyl group is attached.

The term halogen refers to a fluorine, a chlorine, a bromine or an iodine atom.

Fluorine and chlorine are preferred halogen atoms in the framework of the present invention.

The term alkoxy refers to alkoxy radical made up of an oxygen radical bearing a saturated straight or branched chain hydrocarbon radical. Included within the scope of this term are methoxy, ethoxy, propoxy, n-butoxy, isobutoxy, sec-butoxy, t-butoxy, n-pentoxy, isopentoxy, sec-pentoxy, t-pentoxy... and the like.

The step of reacting a compound of formula (III) as defined above, with a compound of formula (IV) as defined above is known as a Suzuki reaction, or Suzuki-Miyaura coupling.

One may refer for example to Suzuki. Orgamet. Chem, 1999, 576, 147-148 *and* Kotha et al., Tetrahedron 58, 2002, 9633-95*.* Said documents are incorporated herein by reference.

As stated in said article, it is worth to remind the advantages of implementing such a reaction. Indeed, it allows to work under gentle conditions. The boronic acid esters are further more easy to handle and are generally thermally stable and insensitive to water and oxygen. The boronic acid esters may also be stored in organic solutions.

Said coupling reaction may be catalyzed by many palladium (0) complexes. Among said catalysts the following may be cited, of expanded formula Pd on carbon, Pd[P(C₆H₅)₃]₄, Pd(OAc)₂, PdCl₂[P(C₆H₅)₃]₂, Pd(PCy₃)₂Cl₂, PdCy₃ where Cy is a cyclohexyl. Other metal catalysts can also be used, including nickel salts and derivatives such as NiCl₂[P(C₆H₅)₃]₂, NiCl₂(PCy₃)₂. According to one embodiment of the invention, the catalyst is chosen among Pd[P(C₆H₅)₃]₄ and Pd(OAc)₂. In the case of Pd(OAc)₂, some ligands can be added such as bulky primary amine. A preferred ligand is adamantamine.

In some cases, aerobic conditions can be used. One may refer to Tao, J. Org. Chem 69, 2004, 4330-35 for an example of aerobic conditions.

Said coupling reaction can moreover be implemented under basic conditions for example in aqueous sodium carbonate solution in dimethoxyethane (DME), in dioxane, in propanone, toluene or in alcohols.

According to further embodiments, other bases such as Et₃N, NaHCO₃, K₃PO₄ and Cs₂CO₃ can be employed.

According to one embodiment of the invention, the coupling reaction is implemented under the following condition, Pd[P(C₆H₅)₃]₄ as catalyst and aqueous Na₂CO₃ in dioxane upon heating under a nitrogen atmosphere.

In the case of sterically hindered boronic acids, strong bases, for example aqueous Ba(OH)₂ or NaOH, accelerate the coupling reaction, while weak bases are more favourable in the case of sterically unhindered boronic acids since hydrolytic deboronation is suppressed. In some cases, anhydrous K₃PO₄ or CsF in aprotic solvents, such as DME, may be used. Organic bases such as tetramethylguanidine (TMG) or 1,8-diazabicyclo[5.4.0]undec-7-ene (DBU) can also be used.

Said coupling stage can be carried out at a temperature of between 70°C and 110 °C. Authors have described related coupling conditions at room temperature but long reaction times are required.

Generally, the molar ratio of the compound of formula (III) to the compound of formula (IV) is preferably between one to one.

When the coupling is carried out under the Suzuki conditions as described above, the molar ratio of the compound of formula (III) and (IV) to the catalyst can be between 3 and 5 % by weight with respect to the compound of formula (III) and (IV).

The solvents which may be used during said coupling step are dioxane ordimethoxyethane.

To a lesser extent, THF or N-methylpyrrolidone, EtOH or EtOH/H₂O can also be used.

Said coupling reaction may for example be carried out over a period of 1 to 6 hours.

Numerous combinaisons of bases, catalysts and sometimes added ligands (e.g.: bulky amines or DABCO for instance in the conditions described by Li in Organic Lett. 6, 2004, 2809-2811) can be employed. Microwave or ultrasound conditions can also be used to reduce reaction times.

The step of reacting a compound of formula (II) as defined above by cyclisation is performed under basic conditions. To this hand conventional basic conditions for such reactions are well known. Among reactants providing said adequate basic reaction sodium amide (NaNH₂) may be cited but also other metal amides such as LiNH₂ or KNH₂. Other bases can be used such metal *tert*-butoxides.

Said step may be carried out in any appropriate solvent such as toluene, dioxane, N-methylpyrrolidone or DMF.

According to one aspect of the invention, said cyclisation step may be carried out in the presence of a catalyst. According to an advantageous aspect the catalyst is a quaternary ammonium or phosphonium salt. It can be chosen among tetrabutylammonium fluoride, chloride or bromide. Triethylbenzylammonium chloride can also be used.

Said cyclisation step may be carried out over a period of 1 to 12 hours. Microwaves or ultrasounds can also be used to accelerate the cyclisation step.

According to an embodiment of the invention, the cyclisation is performed under the following conditions: heating in toluene at 80-100 °C.

The process according to the present invention may be illustrated by the following scheme 1:

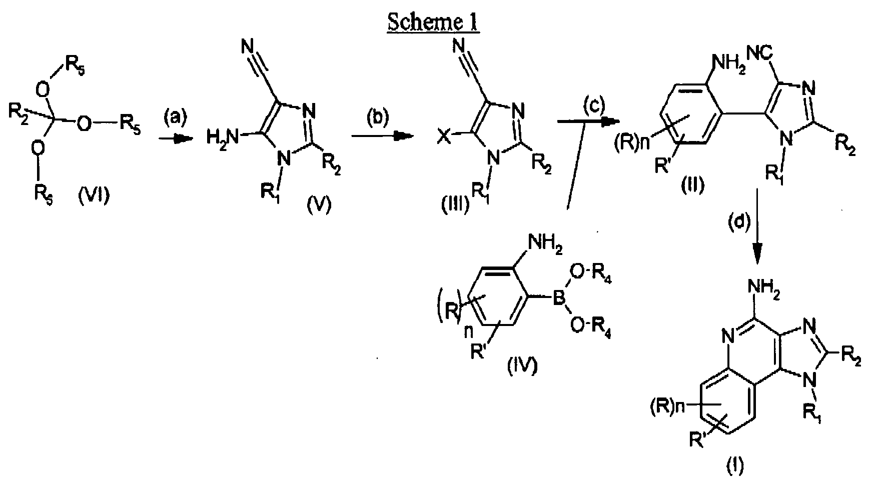

The step (a) may be carried out according to the following scheme 2.

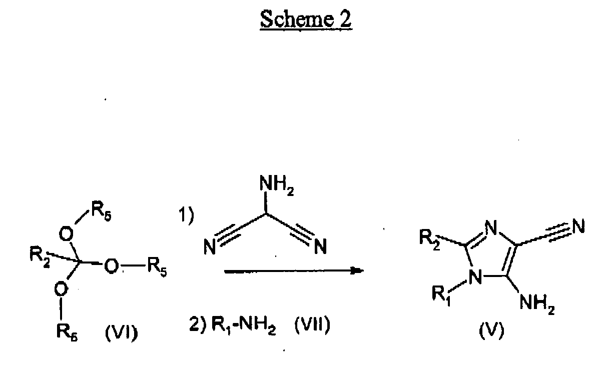

According to scheme 2, aminomalononitrile can be firstly refluxed, in CH₃CN with a compound of formula (VI) where R₂ is as defined above. After 0.5 to 1 hour refluxing, the mixture can be cooled to room temperature and a compound of formula (VII), where R₁ is as defined above at a temperature which can be between 15 and 40°C is stirred for 6-12 hours in order to obtain a imidazole of formula (V). Said reaction step is described in more details in Cristalli G. et al., G. J. Med. Chem. 1996, 34,1188-1192 and Peinador C. et al., M-J. Tetrahedron, 1997, 53, 8269-8272, which are incorporated herein by reference.

The isolated yield can be improved by a modification of the work up, i.e. by direct crystallisation of the crude solid afforded compound of formula (V).

The compound of formula (VI) may be commercially available or may be obtained according to the following scheme 3

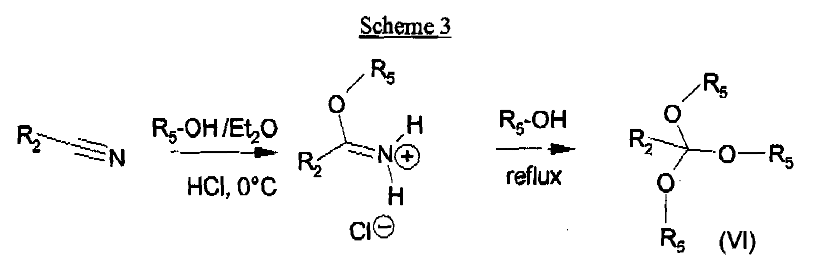

According to said scheme 3, anhydrous gaseous HCl can be added to a solution of nitrile kept at -5 to 0 °C. The solution can be kept below 5°C for 3 to 5 days. The precipitate can be collected and refluxed in a mixture of alcohol R₅OH and cyclohexane to afford (VI).

The compound (V) can be subsequently reacted during step (**b**) with a compound of formula X₂CH₂, where X is as defined above and preferably with diiodomethane. Said reaction may for example be carried out using an organic nitrite as diazotation reagent under known conditions for example described in Minakawa et al, J. Amer Chem. Soc. 2003, 125, 9970-9982., in order to obtain the compound of formula (III). As far as organic nitrites are concerned, isoamylnitrite or tert-butyl nitrite are for example equally efficient.

Thereafter, the compound of formula (II) can be obtained, during step (**c**) by the coupling Suzuki reaction as previously described. Step (**d**) corresponds to the cyclisation step affording the compound of formula (I), according to the reaction conditions as described above.

A "leaving group" can easily be cleaved of a molecule by rupture of heterolitic bindings, with departure of an electronic pair. This group can thus be replaced easily by another group during a substitution reaction for example. Such groups therefore are, for example the halogens, or activated hydroxy groups such as a mesyl, tosyl, triflate, acetyl, etc. Examples of leaving groups as references related to said preparations are given in "Advances Organic Chemistry", J. March, 3rd Edition, Wiley Interscience, p 310-316.

The following scheme 4 illustrates a process of preparation according to the present invention dedicated to the synthesis of Imiquimod,

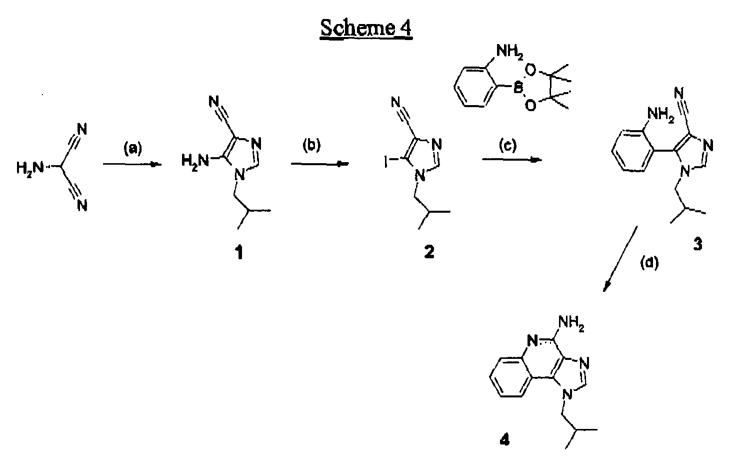

Indeed the present invention also encompasses the process of preparation according to the present invention, wherein the 1H-imidazo[4,5-c]-quinoline ring system is Imiquimod.

According to another aspect, the 1H-imidazo[4,5-c]-quinoline ring system is Resiquimod.

According to scheme 4, in the first step the 5-aminoimidazole-4-carbonitrile 1 can be prepared from aminomalononitrile p-tosylate as previously described by Cristalli for analogous derivatives, for example, in the presence of HC(OEt)₃, during 12 hours at a temperature of 20°C. The 5-aminoimidazole-4-carbonitrile can be converted to the 5-iodoimidazole-4-carbonitrile 2, for example by treatment with isopentyl nitrite in diiodomethane during 1 hour at a temperature of 75°C. The key Suzuki-Myaura coupling can be proceeded smoothly under mild conditions: for example, the reaction can be complete after 1-2 hours at 80°C in the presence of Pd[(C₆H₅)₃], 2N Na₂CO₃ in dioxane. Although both reagent were ortho substituted, the use of an iodide as leaving group and the contribution of an electron-withdrawing nitrile group greatly can facilitate the reaction.

Cyclisation of 3 into imiquimod 4 can be performed under the following basic conditions: Sodium amide in toluene at 80-100°C.

It follows that a simple 4 steps economical synthesis of Imiquimod is encompassed within the present invention. The key feature of the strategy is the Suzuki-Miyaura coupling of the easily obtainable and highly reactive iodoimidazole 2 with a commercially available boronate.

Other preferred embodiments of the final product of formula (I) are given thereafter.

US 4,689,338 discloses a first 1H-imidazo[4,5-c]-quinoline ring systems derivatives family. According to one aspect of the invention, the final product is a compound of formula (I) as defined above, wherein

**R₁** is selected from the group consisting of hydrogen, alkyl of one to about ten carbon atoms, hydroxyalkyl of one to about six carbon atoms, acyloxyalkyl wherein the acyloxy moiety is alkanoyloxy of two to about four carbon atoms or benzoyloxy, and the alkyl moiety contains one to about six carbon atoms, benzyl, (phenyl)ethyl and phenyl, said benzyl, (phenyl)ethyl or phenyl substituent being optionally substituted on the benzene ring by one or two moieties independently selected from the group consisting of alkyl of one to about four carbon atoms, alkyl alkanoate wherein the alkyl moiety contains one to four carbon atoms and the alkanoate moiety contains two to four carbon atoms, alkoxy of one to about four carbon atoms and halogen;

**R₂** is selected from the group consisting of hydrogen, trifluoromethyl, hydroxyalkyl of one to six carbon atoms, aminoalkyl of one to four carbon atoms, mercapto, alkylthio of one to four carbon atoms, alkyl of one to about eight carbon atoms, benzyl, (phenyl)ethyl and phenyl, the benzyl, (phenyl)ethyl or phenyl substituent being optionally substituted on the benzene ring by one or two moieties independently selected from the group consisting of alkyl of one to about four carbon atoms, alkoxy of one to about four carbon atoms and halogen ;

Each **R** is independently selected from the group consisting of alkoxy of one to about four carbon atoms, halogen with the proviso that R is different from a bromine or a iodine atom except when the R group is in the para position to the NH₂ group on the benzene ring and alkyl of one to about four carbon atoms,
**R'** is an hydrogen atom, and
**n** is an integer from 1 to 2,
or a pharmaceutically acceptable acid addition salt thereof.

EP 389 302 discloses a second 1H-imidazo[4,5-c]-quinoline ring systems derivatives family. According to a another aspect of the invention, the final product is a compound of formula (I) as defined above, wherein

**R₁** is selected from the group consisting of straight chain or branched chain alkenyl containing 2 to about 10 carbon atoms and substituted straight chain or branched chain alkenyl containing 2 to about 10 carbon atoms, wherein the substituent is selected from the group consisting of straight chain or branched chain alkyl containing 1 to about 4 carbon atoms, cycloalkyl containing 3 to about 6 carbon atoms and cycloalkyl containing 3 to about 6 carbon atoms substituted by straight chain or branched chain alkyl containing 1 to about 4 carbon atoms;

**R₂** is selected from the group consisting of hydrogen, straight chain or branched chain alkyl containing one to about eight carbon atoms, benzyl, (phenyl)ethyl and phenyl, the benzyl, (phenyl)ethyl or phenyl substituent being optionally substituted on the benzene ring by one or two moieties independently selected from the group consisting of straight chain or branched chain alkyl containing one to about four carbon atoms, straight chain or branched chain alkoxy containing one to about four carbon atoms, and halogen ;
each **R** is independently selected from the group consisting of straight chain or branched chain alkoxy containing one to about four carbon atoms, halogen, with the proviso that R is different from a bromine or a iodine atom except when the R group is in the para position to the NH₂ group on the benzene ring and straight chain or branched chain alkyl containing one to about four carbon atoms,
**R'** is an hydrogen atom, and
**n** is an integer from zero to 2,
or a pharmaceutically acceptable acid addition salt thereof.

WO93/05042 discloses a third 1H-imidazo[4,5-c]-quinoline ring systems derivatives family. According to a another aspect of the invention, the final product is a compound of formula (I) as defined above, wherein
**R₁** is a -CHRaRb group;
**Rb** is hydrogen or a carbon-carbon bond, with the proviso that when **Rb** is hydrogen **Ra** is alkoxy of one to about four carbon atoms, hydroxyalkoxy of one to about four carbon atoms, 1-alkynyl of two to about ten carbon atoms, tetrahydropyranyl, alkoxyalkyl wherein the alkoxy moiety contains one to about four carbon atoms and the alkyl moiety contains one to about four carbon atoms, 2-, 3-, or 4-pyridyl, and with the further proviso that when **Rb** is a carbon-carbon bond **Rb** and **Ra** together form a tetrahydrofuranyl group optionally substituted with one or more substituents independently selected from the group consisting of hydroxy and hydroxyalkyl of one to about four carbon atoms;
**R₂** is selected from the group consisting of hydrogen, alkyl of one to about four carbon atoms, phenyl, and substituted phenyl wherein the substituent is selected from the group consisting of alkyl of one to about four carbon atoms, alkoxy of one to about four carbon atoms, and halogen.
**R** is selected from the group consisting of hydrogen, straight chain or branched chain alkoxy containing one to about four carbon atoms, halogen, with the proviso that R is different from a bromine or a iodine atom except when the R group is in the para position to the NH₂ group on the benzene ring and straight chain or branched chain alkyl containing one to about four carbon atoms;
**R'** is an hydrogen atom, and
**n** is 1;
or a pharmaceutically acceptable acid addition salt thereof.

WO92/15582 discloses a fourth 1H-imidazo[4,5-c]-quinoline ring systems derivatives family. According to a another aspect of the invention, the final product is a compound of formula (I), as defined above, wherein
**R₂** is a-CHX'R'₂R'₃ group;
**R₁** is selected from the group consisting of: hydrogen; straight chain or branched chain alkyl containing one to about ten carbon atoms and substituted straight chain or branched chain alkyl containing one to about ten carbon atoms, wherein the substituent is selected from the group consisting of cycloalkyl containing three to about six carbon atoms and cycloalkyl containing three to about six carbon atoms substituted by straight chain or branched chain alkyl containing one to about four carbon atoms; straight chain or branched chain alkenyl containing two to about ten carbon atoms and substituted straight chain or branched chain alkenyl containing two to about ten carbon atoms, wherein the substituent is selected from the group consisting of cycloalkyl containing three to about six carbon atoms and cycloalkyl containing three to about six carbon atoms substituted by straight chain or branched chain alkyl containing one to about four carbon atoms; hydroxyalkyl of one to about six carbon atoms; alkoxyalkyl wherein the alkoxy moiety contains one to about four carbon atoms and the alkyl moiety contains one to about six carbon atoms; acyloxyalkyl wherein the acyloxy moiety is alkanoyloxy of two to about four carbon atoms or benzoyloxy, and the alkyl moiety contains one to about six carbon atoms; benzyl; (phenyl)ethyl; and phenyl; said benzyl, (phenyl)ethyl, or phenyl substituent being optionally substituted on the benzene ring by one or two moieties independently selected from the group consisting of alkyl of one to about four carbon atoms, alkoxy of one to about four carbon atoms, and halogen ;
**R'₂** and **R'₃** are independently selected from the group consisting of hydrogen, alkyl of one to about four carbon atoms, phenyl, and substituted phenyl wherein the substituent is selected from the group consisting of alkyl of one to about four carbon atoms, alkoxy of one to about four carbon atoms, and halogen;
**X'** is selected from the group consisting of alkoxy containing one to about four carbon atoms, alkoxyalkyl wherein the alkoxy moiety contains one to about four carbon atoms and the alkyl moiety contains one to about four carbon atoms, hydroxyalkyl of one to about four carbon atoms, haloalkyl of one to about four carbon atoms, alkylamido wherein the alkyl group contains one to about four carbon atoms, amino, substituted amino wherein the substituent is alkyl or hydroxyalkyl of one to about four carbon atoms, azido, chloro, hydroxy, 1-morpholino, 1-pyrrolidino, and alkylthio of one to about four carbon atoms; and
**R** is selected from the group consisting of hydrogen, straight chain or branched chain alkoxy containing one to about four carbon atoms, halogen, with the proviso that R is different from a bromine or a iodine atom except when the R group is in the para position to the NH₂ group on the benzene ring and straight chain or branched chain alkyl containing one to about four carbon atoms;
**R'** is an hydrogen atom, and
**n** is 1;
or a pharmaceutically acceptable acid addition salt thereof

US 2004/0147593 discloses a fifth 1H-imidazo[4,5-c)-quinoline ring systems derivatives family. According to another aspect of the invention, the final product is a compound of formula (I) as defined above, wherein
**R₁** and **R₂** are defined as above,
**R'** is selected from the group consisting of:
-Z-Ar,
-Z-Ar'-Y-R₁₁,
-Z-Ar'-W-Y-R₁₁,
-Z-Ar'-R₅, and
-Z-Ar'-W-R₅,

**Ar** is selected from the group consisting of aryl and heteroaryl both of which can be unsubstituted or can be substituted by one or more substituents independently selected from the group consisting of alkyl, alkenyl, alkoxy, methylenedioxy, haloalkyl, haloalkoxy, halogen, nitro, hydroxy, hydroxyalkyl, mercapto, cyano, carboxy, formyl, aryl, aryloxy, arylalkoxy, heteroaryl, heteroaryloxy, heteroarylalkoxy, heterocyclyl, heterocyclylalkyl, amino, alkylamino, and dialkylamino.

**Ar'** is selected from the group consisting of arylene and heteroarylene both of which can be unsubstituted or can be substituted by one or more substituents independently selected from the group consisting of alkyl, alkenyl, alkoxy, haloalkyl, haloalkoxy, halogen, nitro, hydroxy, hydroxyalkyl, mercapto, cyano, carboxy, formyl, aryl, aryloxy, arylalkoxy, heteroaryl, heteroarylox, heteroarylalkoxy, heterocyclyl, heterocyclylalkyl, amino, alkylamino, and dialkylamino.

**W** is selected from the group consisting of alkylene, alkenylene, alkynylene, arylene, heteroarylene, and heterocyclylene wherein the alkylene, alkenylene, and alkynylene groups can be optionally interrupted or terminated with arylene, heteroarylene, or heterocyclylene, and optionally interrupted by one or more -0- groups;

**Y** is selected from the group consisting of:
-S(O)₀₋₂-, -S(O)₂-N(R₈)-, -C(R₆)-,
-C(R₆)-O-, -O-C(R₆)-, -O-C(O)-O-,
-N(R₈)-O-, -C(R₆)-N(R₈)-,
-O-C(R₆)-N(R₈)-, -C(R₆)-N(OR₉)-, 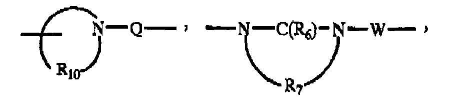 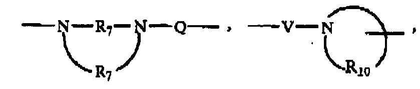 and

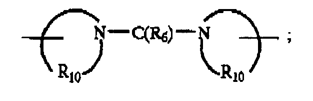

**Z** is selected from the group consisting of a bond, alkylene, alkenylene, and alkynylene.

**R₁₁** is selected from the group consisting of hydrogen, alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl wherein the alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl groups can be unsubstituted or substituted by one or more substituents independently selected from the group consisting of alkyl, alkoxy, hydroxyalkyl, haloalkyl, haloalkoxy, halogen, nitro, hydroxy, mercapto, cyano, aryl, aryloxy, arylalkyleneoxy, heteroaryl, heteroaryloxy, heteroarylalkyleneoxy, heterocyclyl, amino, alkylamino, dialkylamino, (dialkylamino)alkyleneoxy, and in the case of alkyl, alkenyl, alkynyl, and heterocyclyl, oxo.
**R₅** is selected from the group consisting of: 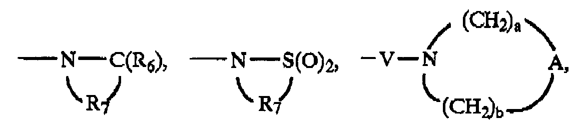 and 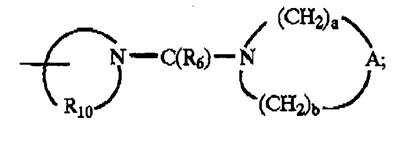 each **R₆** is selected from the group consisting of =O and =S;
each **R₇** is independently C₂₋₇ alkylene;
**R₈** is selected from the group consisting of hydrogen, alkyl, alkoxyalkylenyl, and arylalkylenyl;
**R₉** is selected from the group consisting of hydrogen and alkyl;
each **R₁₀** is independently C₃₋₈ alkylene;
**A** is selected from the group consisting of
-O-, -C(O)-, -S(O)₀₋₂-, -CH₂-, and -N(R₁₁)-;
**Q** is selected from the group consisting of a bond, -C(R₆)-, -C(R)₆-C(R₆)-, -S(O)₂-, -O(R₆)-N(R₈)-W'-, -S(O)₂-N(R₈)-, -C(R₆)-O-, and - C (R₆)-N(OR₉)-;
**V** is selected from the group consisting of -C(R₆)-, -O-C(R₆)-, - N(R₈)-C(R₆)-, and -S(O₂)-;
**W'** is selected from the group consisting of a bond, -C(O)-, and -S(O)₂-; and
a and b are independently integers from 1 to 6 with the proviso that a+b is ≦7;
the potential other R substituant is as defined above and n is 1 or 2.
or a pharmaceutically acceptable acid addition salt thereof.

WO 2006/091567 discloses a sixth 1H-imidazo[4,5-c]-quinoline ring systems derivatives family.

According to another aspect of the invention, the final product is a compound of formula (1) as defined above, wherein
**R₁** is selected from the group consisting of:
-R₁₁,
-W-R₁₁,
-W-Y-R₁₁, and
-W-R₅;
R₂ is -CH₂OH of -(CH₂)₂OH,
**R'** is preferably on the para position of the phenyl group and is selected from the group consisting of: -Z-Ar, -Z-Ar'-Y-R₁₁, and -Z-Ar'-W-Y-R₁₁;
**Ar** is selected from the group consisting of aryl and heteroaryl both of which can be unsubstituted or can be substituted by one or more substituents independently selected from the group consisting of alkyl, alkenyl, alkoxy, haloalkyl, haloalkoxy, halogen, nitro, hydroxy, hydroxyalkyl, mercapto, cyano, carboxy, formyl, amino, alkylamino, and dialkylamino;

**Ar'** is selected from the group consisting of arylene and heteroarylene both of which can be unsubstituted or can be substituted by one or more substituents independently selected from the group consisting of alkyl, alkenyl, alkoxy, haloalkyl, haloalkoxy, halogen, nitro, hydroxy, hydroxyalkyl, mercapto, cyano, carboxy, formyl, amino, alkylamino, and dialkylamino;
**W** is alkylene optionally interrupted by one -0- group;
**Y** is selected from the group consisting of:
-O-,
-C(R₆)-,
-C(R₆)-N(R₈)-,
-S(O)₀₋₂-,
-N(R₈)-Q-, 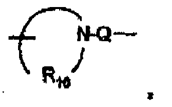 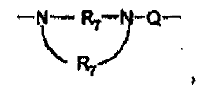 and 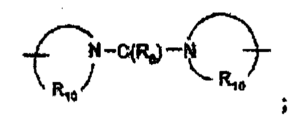 and
**Z** is selected from the group consisting of a bond and alkylene;
**R₁₁** is selected from the group consisting of hydrogen, alkyl, alkenyl, aryl, arylalkylenyl, heteroaryl, heteroarylalkylenyl, and heterocyclyl, wherein the alkyl, alkenyl, aryl, arylalkylenyl, heteroaryl, heteroarylalkylenyl, and heterocyclyl groups can be unsubstituted or substituted by one or more substituents independently selected from the group consisting of alkyl, alkoxy, hydroxyalkyl, haloalkyl, haloalkoxy, halogen, nitro, hydroxy, mercapto, cyano, aryl, aryloxy, heteroaryl, heteroaryloxy, heterocyclyl, amino, alkylamino, dialkylamino, and, in the case of alkyl, alkenyl, and heterocyclyl, oxo;
**R₅** is selected from the group consisting of: 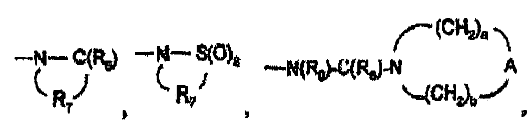 and 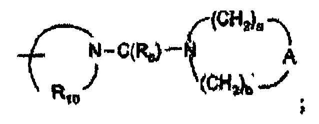 **R₆** is selected from the group consisting of =O and =S;
**R₇** is C₂₋₇ alkylene;
**R₈** is selected from the group consisting of hydrogen, alkyl, alkoxyalkylenyl, hydroxyalkylenyl, arylalkylenyl, and heteroarylalkylenyl;
each **R₁₀** is independently C₃₋₈ alkylene;
**A** is selected from the group consisting of -O-, -C(O)-, -CH₂-, -S(O)₀₋₂-, and -N(Q-R₁₁)-;
**Q** is selected from the group consisting of a bond, -C(R₆)-, -S(O)₂, -C(R₆)-N(R₈) -, -S(O)₂-N(R₈) -, -C(R₆)-O-, and -C(R₆)-S-; and
**a** and **b** are independently integers from 1 to 6 with the proviso that a + b is ≤7;
**R** is an hydrogen atom, and
**n** is 1,
or a pharmaceutically acceptable acid addition salt thereof.

The following examples illustrate the preparation process according to the invention. The NMR analyses confirm the structures of the compounds obtained. NMR spectra were obtained on a Bruker 400 MHz spectrometer.

### Example 1 : synthesis of Imiquimod

The synthesised compounds are numbered according to scheme 4 as reported above.

### Step (a)

### 5-Amino-1-(2-methylpropyl)imidazole4-carbonitrile (1).

Anhydrous ammonia was passed, at room temperature, through a stirred suspension of aminonitrile tosylate (10 g, 39 mmol) in 200 mL CH₃CN. After 45 min, ammonia was stopped and the solution was cooled at 15°C. The solid was filtered off and washed once with 10 mL CH₃CN, The filtrate was combined with the washing and concentrated to half of its volume. To this solution, triethyl orthoformate (6.6 mL, 39.5 mmol) was added and the solution was refluxed 30 min. After cooling to r.t, 2-methylpropylamine (*iso*-butylamine) (3.95 mL, 39.5 mmol) was added and the mixture was stirred overnight at r.t. The solution was evaporated to dryness *in vacuo* and the residue was partitioned between CH₂Cl₂ and 2N Na₂CO₃. The organic layer was evaporated and the residue was triturated in 20 mL AcOEt. The brown crystals that separated were separated and could be used for the next step.

Yield: 50-60 %. An analytical sample was prepared by crystallisation from AcOEt.; Mp: 193-195°C. ¹H-NMR (400 MHz, CDCl₃) δ 0.91 (d, 6H, 2CH₃); 1.98 (non, 1H, CH); 3.50 (d, 2H, CH₂); 3.83 (br s, 2H, NH₂); 6.97 (s, 1H, imidazole). ¹³C-NMR (100 MHz, CDCl₃) 20.24; 29.38; 51.96; 116.11; 113.89.

### Step (b)

### 5-Iodo-1-(2-methylpropyl)imidazole-4-carbonitrile (2).

To a hot (80°C) suspension of compound 1 (3.28 g, 20 mmol) in CH₂I₂ (100 mL) isoamylnitrite (isopentylnitrite) (4.68 g , 40 mmol) was added dropwise over 30 mn Stirring was pursued 1 h at the same temperature. Diiodomethane was then recovered as a pink oil by distillation *in vacuo* Bp(10 mm Hg): 90-100 °C. The residue was taken up with AcOEt and crystallized upon trituration with diethyl ether: Mp: 174-176°C.

Yield 80-85 % ¹H-NMR (400 MHz, CDCl₃) δ: 0.75 (d, 6H, 2 CH₃); 1.96 (non, 1H, CH); 3.55 (d, 2H, CH₂); 7.42 (1H, s, imidazole).

### Step (c)

### 5-(2-Aminophenyl)-1-(2-methylpropyl)imidazole-4-carbonitrile (3).

A slow bubbling of nitrogen was maintained throughout the reaction. To a solution of compound 2 (2.75, 10 mmol) in 20 mL dioxane was added 10 mL 2N Na₂CO₃, Pd[(P(C₆H₅)₃]₄ (0.55 g, 0.5 mmol) and pinacol 2-(aminophenyl)boronate (2.80, 10 mmol). The mixture was stirred 2h at 80 °C. After cooling to r.t., the mixture was concentrated *in vacuo* to half of its initial volume and then extracted twice with 20 mL AcOEt. The organic layer was dried over Na₂SO₄ and evaporated. The residue was triturated with 2 mL AcOEt to afford 3.

Yield: 85-95 %. Mp : 145-150°C. ¹H-NMR(400 MHz, CDCl₃) δ 0.78 and 0.80 (2d, 2x3H, 2 CH₃); 1.80(non, 1H, CH) 3.60(broad s, 2H, NH₂); 3.65(d, 2H, CH₂); 6.80(, 1H, Aro); 6.83(t, 1H, Aro); 7.30(t, 1H, Aro); 7.60(s, 1H, imi). IR(KBr): ν 748; 1449; 1643; 2229; 2955; 3440; 3741 cm¹.

### Step (d)

### 4-Amino-1-(2-methylpropyl)-imidazo-[4,5-c]quinoline (Imiquimod) (4).

To compound 3, (1.20, 5 mmol), suspended in 20 mL of anhydrous toluene, was added under N₂ at room temperature (0.2 g, 5 mmol) NaNH₂. The mixture was stirred at 90-100°C. Monitoring of the reaction by thin layer chromatography showed complete conversion in less than 2h. After cooling below 5°C 2 mL of ice water were added dropwise and the mixture was evaporated until dryness. Imiquimod was isolated upon trituration of the residue with 5 mL cold water.

Yield: 90-96% Mp > 260°C. ¹H-NMR (400 MHz, CDCl₃) 1.02(d, 6H, 2 CH₃); 2.35(non, 1H, CH); 4.30(d, 2H, CH₂); 5.50(s, 2H, NH₂); 7.32(t, 1H, 8-H), 7.55(t, 1H, 7-H); 7.80(s, 1H, 2-H); 7.82(d, 1H, 6-H); 7.92(d, 1H, 9-H).

## Claims

1. Compound of formula (II) 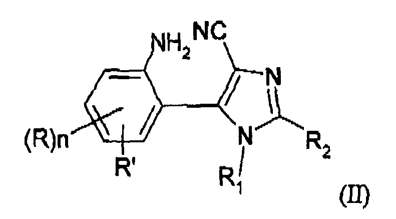 wherein
**R₁** represents:
- an hydrogen atom,
- a (C₁-C₁₀)alkyl group,
- a phenyl group,
- a benzyl group,
- a (pltenyl)ethyl group,
- a (C₂-C₁₀)alkenyl group,
- a tetrahydrofuranyl methyl group,
- R₁₁,
- W-R₁₁,
- W-Y-R₁₁, or
- W-R₅,
where the (C₁-C₁₀)alkyl group can possibly be substituted by one or two identical or different groups chosen among an hydroxy group, a (C₁-C₄)alkoxy group, a hydroxy (C₁-C₄)alkoxy group, a (C₃-C₆)cycloalkyl group, a (C₂-C₄)alkanoyloxy(C₁-C₆)alkyl group, a benzoyloxy(C₁-C₆)alkyl group, a (C₂-C₁₀)alk-1-ynyl group, a tetrahydropyranyl group, a (C₁-C₄)alkoxy(C₁-C₄)alkyl group or a 2-, 3- or 4-pyridyl group,
where the (C₂-C₁₀)alkenyl group can be possibly substituted by one or two identical or different groups chosen among a (C₁-C₄)alkyl group, a (C₃-C₆)cycloalkyl group optionally substituted by a (C₁-C₄)alkyl group, and
where the tetrahydrofuranyl group can be possibly substituted by one substituent selected among a hydroxy group and a (C₁-C₄)hydroxyalkyl group,
**R₂** represents:
- an hydrogen atom,
- a trifluoromethyl group,
- a (C₁-C₈)alkyl group,
- a mercapto group,
- a (C₁-C₄)alkylthio group,
- a benzyl group,
- a (phenyl)ethyl group,
- a phenyl group,
where the (C₁-C₈)alkyl group can possibly be substituted by one or three identical or different groups chosen among a phenyl group, a (C₁-C₄)alkoxy group, a (C₁-C₄)alkoxy(C₁-C₄)alkyl group, a hydroxy(C₁-C₄)alkyl group, a halo(C₁-C₄)alkyl group, a (C₁-C₄)alkylamido, an amino group or an amino group substituted independently by a (C₁-C₄)alkyl group or a hydroxy(C₁-C₄)alkyl group, an azide group, a chlorine atom, a hydroxy group, a 1-morpholino group, a 1-pyrrolidino group or a (C₁-C₄)alkylthio group,
where the phenyl group of the benzyl, the (phenyl)ethyl or the phenyl group in R₁ or R₂ can be possibly substituted by one or two substituents selected from the group consisting of a (C₁-C₄)alkyl group, a (C₁-C₄)alkoxy group, a (C₁-C₄)alkyl(C₂-C₄)alkoxy group or a halogen atom,
**R** independently represents:
- a (C₁-C₁₀)alkoxy group,
- an halogen group, with the proviso that R is different from a bromine or a iodine atom except when the R group is in the para position to the NH₂ group on the benzene ring,
- a (C₁-C₄)alkyl group,
- a hydroxy group, or
- a trifluoromethyl group,
n is an integer from 0 to 2,
**R'** represents H or when n is 0 or 1 **R'** is H or may be selected from the group consisting of:
- Z-Ar,
- Z-Ar'-Y-R₁₁,
- Z-Ar'-W-Y-R₁₁,
- Z-Ar'-R₅, and
- Z-Ar'-W-R₅;
where Ar is selected from the group consisting of aryl and heteroaryl both of which can be unsubstituted or can be substituted by one or more substituents independently selected from the group consisting of alkyl, alkenyl, alkoxy, methylenedioxy, haloalkyl, haloalkoxy, halogen, nitro, hydroxy, hydroxyalkyl, mercapto, cyano, carboxy, formyl, aryl, aryloxy, arylalkoxy, heteroaryl, heteroaryloxy, heteroarylalkoxy, heterocyclyl, heterocyclylalkyl, amino, alkylamino, and dialkylamino;
**Ar'** is selected from the group consisting of arylene and heteroarylene both of which can be unsubstituted or can be substituted by one or more substituents independently selected from the group consisting of alkyl, alkenyl, alkoxy, haloalkyl, haloalkoxy, halogen, nitro, hydroxy, hydroxyalkyl, mercapto, cyano, carboxy, formyl, aryl, aryloxy, arylalkoxy, heteroaryl, heteroarylox, heteroarylalkoxy, heterocyclyl, heterocyclylalkyl, amino, alkylamino, and dialkylamino;
**W** is selected from the group consisting of alkylene, alkenylene, alkynylene, arylene, heteroarylene, and heterocyclylene wherein the alkylene, alkenylene, and alkynylene groups can be optionally interrupted or terminated with arylene, heteroarylene, or heterocyclylene, and optionally interrupted by one or more -0- groups;
**Y** is selected from the group consisting of:
- O-
- S(O)₂-, -S(O)₂-N(R₅)-, -O(R₆)-,
- O(R₆)-O- -O-O(R₅)-, -O-C(O)-O-,
- N(R₆)-O-, -C(R₅)-N(R₈)-,
- O-C(R)-N(R)-, -C(R₆)-N(OR₈)-, 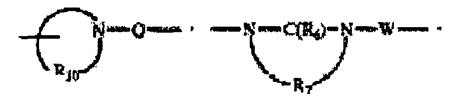 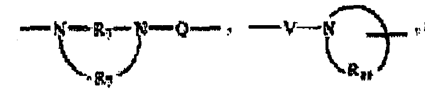 and 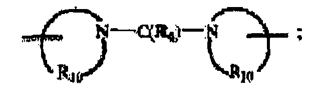
**Z** is selected from the group consisting of a bond, alkylene, alkenylene, and alkynylene;
**R₁₁** is selected from the group consisting of hydrogen, alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroaryienyl, and heterocyclyl wherein the alkyl, alkenyl, alkynyl, aryl, arylalkylenyl, aryloxyalkylenyl, alkylarylenyl, heteroaryl, heteroarylalkylenyl, heteroaryloxyalkylenyl, alkylheteroarylenyl, and heterocyclyl groups can be unsubstituted or substituted by one or more substituents independently selected from the group consisting of alkyl, alkoxy, hydroxyalkyl, haloalkyl, haloalkoxy, halogen, nitro, hydroxy, mercapto, cyano, aryl, aryloxy, arylalkyleneoxy, heteroaryl, heteroaryloxy, heteroarylalkyleneoxy, heterocyclyl, amino, alkylamino, dialkylamino, (dialkylamino)alkyleneoxy, and in the case of alkyl, alkenyl, alkynyl, and heterocyclyl, oxo;
**R₅** is selected from the group consisting of: 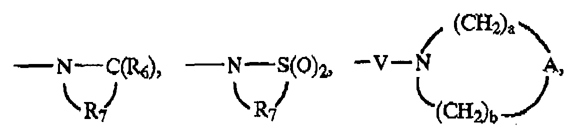 and 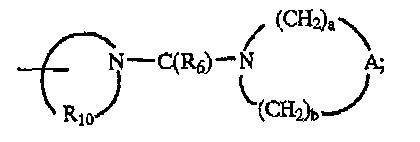 each **R₆** is independently selected from the group consisting of =O and =S;
each **R₇** is independently C₂₋₇ alkylene;
**R₈** is selected from the group consisting of hydrogen, alkyl, alkoxyalkylenyl, hydroxyalkylenyl, heteroarylalkylenyl and arylalkylenyl;
**R₉** is selected from the group consisting of hydrogen and alkyl;
each **R₁₀** is independently C₃₋₈ alkylene;
**A** is selected from the group consisting of -O-, -C(O)-, -S(O)₀₋₂-, -N(Q-R₁₁)-, -CH₂-, and -N(R₁₁)-;
**Q** is selected from the group consisting of a bond, -C(R₆)-, -C(R₆)-C(R₆)-, -S(O)₂-, -C(R₆)-N(R₈)-W'-, -S(O)₂-N(R₈)-, -C(R₆)-O-, -C(R₆)-S- and -C(R₆)-N(OR₉)-;
V is selected from the group consisting of -C(R₆) -, -O-C(R₆)-, -N(R₈-C(R₆) -, and -S(O)₂-;
W' is selected from the group consisting of a bond, -C(O) -, and -S(O)₂-; and
a and b are independently integers from 1 to 6 with the proviso that a+b is ≤7;
or a pharmaceutically acceptable acid addition salt thereof

2. Compound of formula (II) according to claim 1 wherein
**R₁** represents
- an hydrogen atom,
- a (C₁-C₁₀) alkyl group,
- a (C₁-C₆) hydroxyalkyl group,
- a acyloxyalkyl group wherein the acyloxy moiety is alkanoyloxy of two to about four carbon atoms or benzoyloxy, and the alkyl moiety contains one to about six carbon atoms,
- a benzyl group,
- a (phenyl)ethyl group,
- a phenyl group,
where the phenyl group of the benzyl, the (phenyl)ethyl or the phenyl group can be possibly substituted by one or two substituents selected from the group consisting of a (C₁-C₄)alkyl group, a (C₁-C₄)alkoxy group, a (C₁-C₄)akyl(C₂-C₄)alkoxy group or a halogen atom,
**R₂** represents
- an hydrogen atom,
- a (C₁-C₈)alkyl group,
- a benzyl group,
- a (phenyl)ethyl group,
- a phenyl group,
where the phenyl group of the benzyl, the (phenyl)ethyl or the phenyl group can be possibly substituted by one or two substituents selected from the group consisting of a (C₁-C₄)alkyl group, a (C₁-C₄)alkoxy group, or a halogen atom,
**R** independently represents
- a (C₁-C₄)alkoxy group,
- an halogen group, with the proviso that R is different from a bromine or a iodine atom except when the R group is in the para position to the NH₂ group on the benzene ring,
- a (C₁-C₄)akyl group,
**R'** is an hydrogen atom, and
n is an integer from 1 to 2,
or a pharmaceutically acceptable acid addition salt thereof.

3. Compound of formula (II) as defined in anyone of the preceding claims for use as a synthetic intermediate.

4. Process for the preparation of a compound of formula (II) as defined in anyone of the preceding claims, wherein it comprises at least the step of reacting a compound of formula 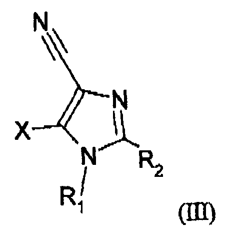 wherein R₁, R₂ are as defined in claim 1 or 2, and X is a diazonium group, triflates or an halogen atom and preferably a bromine or a iodine atom, by coupling, in the presence of a catalyst, with a compound of formula 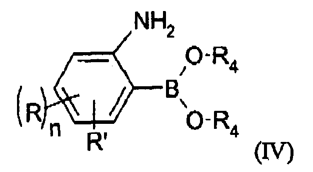 wherein R, R' and n are as defined in claim 1 or 2, and
wherein R₄ represents an hydrogen atom, an alkyl group and where the two R₄ groups can also be bound together to form a 5- or 6-membered ring 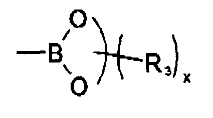 where represents a (C₂-C₄)alkylene possibly substituted, x is an integer from 1 to 4 and R₃ independantly represents an hydrogen atom or a (C₁-C₄)alkyl group.

5. Process according to claim 4, wherein the coupling reaction is performed with a catalyst chosen among palladium (O) complexes such as Pd on carbon or by nickel salts.

6. Process according to the preceding claim, wherein the catalyst is chosen among Pd[P(C₆H₅)₃]₄, Pd(OAc)₂, PdCl₂[P(C₆H₅)₃]₂C, Pd(PCy₃)₂Cl₂, PdCy₃ where Cy is a cyclohexyl and among NiCl₂[P(C₆H₅)₃]₂, and NiCl₂(PCy₃)₂.

7. Process according to anyone of claims 5 or 6, wherein the catalyst is chosen among Pd[P(C₆ H₅)₃]₄ and Pd(OAc)₂ with optionally some other ligands such as bulky primary amine and preferably the ligand adamantine.

8. Process according to anyone of claims 4 to 7, wherein said coupling reaction is implemented under basic conditions such as in aqueous sodium carbonate solution in dimethoxyethane, in dioxane, in propanone, toluene or in alcohols or thanks to other bases chosen among Et₃N, NaHCO₃, K₃PO₄ and Cs₂ CO₃.

9. Process according to anyone of claims 4 to 8, wherein said coupling reaction is carried out at a temperature of between 70°C and 110°C.

10. Process for the preparation of preparing 1H-imidazo[4,5-c]-quinoline ring systems derivatives of formula (I) 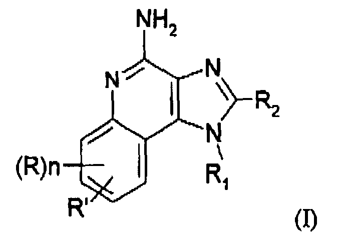 where Rt, R₂, R, R' and n are as defined in anyone of claims 1 or 2,
comprising the step of reacting a compound of formula (II) as defined in anyone of claims 1 or 2, by cyclisation under basic conditions.

11. Process according to claim 10, wherein said basic conditions are provided by sodium amide [NaNH₂], by other metal amides chosen among LiNH₂ and KNH₂, or by metal *tert*-butoxides.

12. Process according to claim 10 or 11, wherein the 1H-imidazo[4,5-c]-quinoline ring systems derivatives of formula (I) is Imiquimod or Resiquimod.
